# EUROPEAN PATENT APPLICATION

(11) **EP 4 699 625 A1**
(43) Date of publication of application: **25.02.2026**
(21) Application number: 24791860.0
(22) Date of filing: 02.04.2024
(51) Int. Cl.: A61L 29/06, A61F 9/007, A61M 1/00

(54) **OPHTHALMIC DRAINAGE DEVICE AND PREPARATION METHOD THEREFOR**

(30) Priority: 20.04.2023 CN 202310425282
(71) Applicant: Health Guard (Suzhou) Biomed. Technology Co., Ltd, Suzhou, Jiangsu 215123 (CN)
(72) Inventor: HAN, Ziming, Suzhou, Jiangsu 215123 (CN); YE, Sai, Suzhou, Jiangsu 215123 (CN); LIU, Zhizhong, Suzhou, Jiangsu 215123 (CN)
(74) Representative: Boult Wade Tennant LLP
(86) International application number: PCT/CN2024/085490
(87) International publication number: WO 2024/217275

(57) **Abstract**

An ophthalmic drainage device and a preparation method therefor. The ophthalmic drainage device comprises a tube wall and a drainage channel formed by the wrapping of the tube wall, wherein the tube wall comprises a biocompatible material; the biocompatible material comprises a polymer that contains an ether group, an ester group and an imino group, and the average crosslinking degree of the biocompatible material in the ophthalmic drainage device is 60%-90%; and the relative density of the ophthalmic drainage device is 40%-60%. The prepared ophthalmic drainage device has a more compact network structure, stronger anti-pressure capability during use, good bending axial pushing behavior and stronger anti-shearing capability, is not prone to experiencing cracking and dissolving in a solution, and is more stable.

## Description

### CROSS REFERENCE TO RELATED APPLICATION

This application claims priority to Chinese Patent Application No. 202310425282.9, filed on April 20, 2023 and entitled "OPHTHALMIC DRAINAGE DEVICE AND PREPARATION METHOD THEREFOR", which is hereby incorporated by reference in its entirety.

### TECHNICAL FIELD

The present application belongs to the technical field of ophthalmic medical biomaterials, and particularly, relates to an ophthalmic drainage device and a preparation method for the same.

### BACKGROUND

Hydrogels com posed of natural polymer materials and derivatives thereof can well simulate human tissue structures due to their high water content, remarkable biomimetic properties, and similar physical properties to extracellular matrix (ECM), and therefore, have been extensively applied in tissue engineering and biomedical fields.

In the related art, when single-network hydrogels with simple structures are prepared into related devices such as ophthalmic drainage devices that are implanted in patients, the devices are often subjected to stress deformation in vivo, and are prone to rupture, leakage, or dissolution. Consequently, such devices fail to remain stable for a long time. Therefore, it is urgent to improve the ophthalmic drainage devices.

### SUMMARY

In view of this, the present application provides an ophthalmic drainage device, a preparation method for the same, and application thereof, aiming to provide an ophthalmic drainage device that facilitates the determination of its intraocular position.

In a first aspect, some embodiments of the present application provide an ophthalmic drainage device, including: a tube wall and a drainage channel defined by the tube wall, where the tube wall includes a biocompatible material including a polymer containing an ether group, an ester group, and an imino group; the biocompatible material in the ophthalmic drainage device has an average cross-linking degree of 60% to 90%; and in a 25°C anhydrous ethanol solution, the ophthalmic drainage device has a relative density of 40% to 60%.

In some embodiments of the present application, the biocompatible material in the ophthalmic drainage device contains 0.25 to 0.4 micromole of the imine group per milligram.

In a second aspect, some embodiments of the present application provide a method for preparing an ophthalmic drainage device, including: placing a raw drainage tube including a core mold in a first organic solution containing a monomer containing an aldehyde group, so that an amino group in the drainage tube is enabled to react with the aldehyde group to obtain a first drainage tube, where the raw drainage tube includes a tube wall and a drainage channel defined by the tube wall, the tube wall includes a multifunctional polymer containing hydrophilic groups and the amino group, and the hydrophilic groups include a carboxyl group and a hydroxyl group; and placing the first drainage tube in a second alkaline organic solution containing a monomer containing an epoxy group, so that the reaction of the epoxy group is enabled to obtain the ophthalmic drainage device in the first aspect.

In some embodiments of the present application, the preparation method includes at least one of the following conditions:
the multifunctional polymer containing the hydrophilic groups and the amino group includes one or both of type A gelatin and type B gelatin;
the monomer containing the aldehyde group includes at least one of glutaraldehyde, oxidized glucan, glyoxal, malondialdehyde, butanedial, hexanedial/o-phthalaldehyde, isophthalaldehyde, terephthalaldehyde, 2,3-naphthalenediacetal 2,5-diformylfuran, 2,5-dimethoxybenzene-1,4-dialdehyde, 4-tert-butyl-2,6-formylphenol, biphenyl-2,2-dialdehyde, 1,4-cyclohexanedimethanol glycidyl ether, resorcinol diglycidyl ether, pentaerythritol glycidyl ether, or neopentyl glycol diglycidyl ether;
the monomer containing the epoxy group includes at least one of polyethylene glycol diglycidyl ether, ethylene glycol diglycidyl ether/terminal epoxy modified polyethylene glycol, terminal amino modified polyethylene glycol or ethylene glycol, aminated glucan, 1,4-butanediol glycidol, or diglycidyl ether;
the first organic solution and the second alkaline organic solution each include at least one of acetonitrile, ethanol, methanol, or tetrahydrofuran; or
the second alkaline organic solution includes at least one of N,N-diisopropylethylamine, triethylamine, pyridine, sodium hydroxide, or potassium hydroxide.

In some embodiments of the present application, the monomer containing the epoxy group has an epoxy value of 0.35 to 0.91 eq/100 g.

In some embodiments of the present application, after a first drainage tube containing a ketone group is obtained, the preparation method further includes: removing the unreacted monomer containing the aldehyde group.

In some embodiments of the present application, after the first drainage tube is placed in a second alkaline organic solution containing a monomer containing an epoxy group, so that the carboxyl group is enabled to react with the epoxy group, the preparation method includes: removing the unreacted monomer containing the epoxy group.

Compared with the prior art, the present application has at least the following beneficial effects:
According to the ophthalmic drainage device provided in the present application, its tube wall is made of the biocompatible material, and the biocompatible material includes the polymer containing the ether group, the ester group, and the imino group; the biocompatible material has the average cross-linking degree of 60% to 90%; and the ophthalmic drainage device has the relative density of 40% to 60% in the 25°C anhydrous ethanol solution. The high cross-linking degree and density enable the tube wall of the ophthalmic drainage device to have a compact network structure. When in use, the ophthalmic drainage device with the compact network structure and the high cross-linking degree is less prone to rupture or dissolution in a short time and more stable.

The preparation method of the present application demonstrates that the ophthalmic drainage tube with fewer reactive functional groups is more stable in structure and performance and does not leak in a short time.

### BRIEF DESCRIPTION OF THE DRAWINGS

In order to explain the technical solutions of the embodiments of the present application more clearly, the accompanying drawings required for use in the embodiments of the present application will be briefly introduced below. Apparently, the accompanying drawings described below show only some embodiments of the present application. A person of ordinary skill in the art may derive other drawings based on these drawings without any creative efforts.
FIG. 1 shows a microstructure of an ophthalmic drainage device in Comparative Example 2 of the present application; and
FIG. 2 shows a microstructure of an ophthalmic drainage device in Embodiment 1 of the present application.

### DETAILED DESCRIPTION

In order to make the objectives, technical solutions, and beneficial technical effects of the present application clearer, the present application will be further explained in detail below in conjunction with the embodiments. It should be understood that the embodiments described in this specification are merely for the purpose of explaining the present application, but not for limiting the present application.

For the sake of simplicity, the present application merely explicitly discloses some numerical ranges. However, any lower limit may be combined with any upper limit to form an unspecified range; any lower limit may be combined with other lower limits to form an unspecified range, and any upper limit may be combined with any other upper limit to form an unspecified range. Moreover, although not explicitly specified, each point or single value between the endpoints of the range is included in the range. Therefore, each point or single value may be combined with any other point or single value as its own lower limit or upper limit or combined with other lower limit or upper limit to form an unspecified range.

In the description of the present application, it should be noted that unless otherwise specified, "above" and "below" refer to that value, and "more" in "one or more" means two or more.

The above summary of the present application is not intended to describe every disclosed embodiment or every implementation in the present application. The following description illustrates exemplary embodiments more specifically. In many places throughout the application, guidance is provided through a series of embodiments, which can be used in various combinations. In each embodiment, the enumeration is merely a representative group and should not be interpreted as exhaustive.

Glaucoma is an irreversible blinding eye disease, characterized primarily by optic disc damage, diminution of vision, and associated visual field defects. The primary manifestations of glaucoma are characterized by pathological elevation of intraocular pressure and insufficient blood supply to the optic nerve. The factors that induce glaucoma are relatively complex.

Currently, the primary clinical approach for treating glaucoma is to lower intraocular pressure. Drainage tubes are often used to lower intraocular pressure. The drainage tube, which is extremely fine, is delivered to an appropriate site via an injector in a minimally invasive manner, and functions by diverting aqueous humor from the anterior chamber to lower intraocular pressure. However, in practical use, once implanted, the drainage tube is subjected to stress and is deformed within the ocular tissues. Moreover, under the influence of various intraocular factors, the drainage tube often fails to remain stable for a long time and is prone to rupture or dissolution, thereby causing inconvenience to patients.

Based on this, the inventor conducted extensive research aimed at providing an ophthalmic drainage device that has a higher relative density and a more stable structure and is less prone to rupture or dissolution.

### Ophthalmic drainage device

In a first aspect, some embodiments of the present application provide an ophthalmic drainage device, including: a tube wall and a drainage channel formed by wrapping the tube wall, where the tube wall includes a biocompatible material, and the biocompatible material includes a polymer containing an ether group, an ester group, and an imino group; the biocompatible material in the ophthalmic drainage device has an average cross-linking degree of 60% to 90%; and the ophthalmic drainage device has a relative density of 40% to 60% in a 25°C anhydrous ethanol solution.

In the related art, ophthalmic drainage devices, which have single material structures, often cannot withstand excessive stress and deformation, thereby compromising use in patients.

Studies have revealed that the biocompatible material, including ether, ester, and imine groups, undergoes compact cross-linking to form a more compact network structure, making it less prone to rupture or dissolution during long-time use; the biocompatible material contains more non-reactive functional groups, such as ether, ester, and imine groups, and less reactive groups, such as amino, carboxyl, and epoxy groups, providing the ophthalmic drainage device with an appropriate in vivo service life, making the drainage device less prone to rupture and dissolution in a short time, and improving the stability of the drainage tube; the low content of functional groups such as carboxyl results in a neutral pH value of the material; and the cross-linking imparts more suitable mechanical properties to the drainage device, such as compressive strength and extensibility.

According to the embodiments of the present application, the biocompatible material may be understood as a copolymer composed of a multifunctional monomer containing hydrophilic groups and an amino group, a monomer containing an aldehyde group, and a monomer containing an epoxy group, where the hydrophilic groups include a hydroxyl group and a carboxyl group, the amino group in the multifunctional monomer may react with the aldehyde group, the epoxy group may react with a carboxylic acid, and the carboxyl group and the hydroxyl group in the hydrophilic groups may react, whereby the biocompatible material having the average cross-linking degree within the above range can be obtained, and the ophthalmic drainage device has a more compact network structure and is less prone to rupture or dissolution in a short time and more stable.

In some implementations, the relative density of the ophthalmic drainage device in the 25°C anhydrous ethanol solution is greater than or equal to 40% and reaches up to 60%, indicating that the network cross-linking density of the ophthalmic drainage tube is lower, which improves the quality of the ophthalmic drainage device. In addition, the high relative density can enhance the user's comfort and drainage effect. As an example, a testing method for the relative density includes: accurately measuring two side lengths and a thickness of an ophthalmic drainage tube sample with a vernier caliper, calculating a volume of a stent (V1), weighing the sample (W1), immersing the sample in the anhydrous ethanol solution until saturation, taking out the sample, and weighing the sample (W2), where the density of anhydrous ethanol is denoted as ρ, and the relative density of the ophthalmic drainage tube P (%) = (W2-W1)/ (ρ • V1) × 100%.

The relative density of the ophthalmic drainage device within the above range indicates that the network density of the material is higher, gaps between materials are smaller, the material is less prone to rupture or dissolution, and the biocompatible material contains less carboxyl group after the carboxyl group reacts with the epoxy group or hydroxyl group.

In some embodiments, the average cross-linking degree of the biocompatible material in the ophthalmic drainage device is 60% to 90%. As an example, a testing method for the cross-linking degree may be a biuret reagent kit method: taking 1 mg of a cross-linked sample to measure its carboxyl content M1 with a reagent kit, taking 1 mg of a sample before cross-linking (first drainage tube) to measure its carboxyl content M2 with a reagent kit, and obtaining the cross-linking degree (M1 - M2)/ M1.

In some embodiments, the biocompatible material in the ophthalmic drainage device contains 0.25 to 0.4 micromole of the imine group per milligram.

According to the embodiments of the present application, the imine functional group per unit mass of the biocompatible material within the above range indicates that the network structure in the tube wall composed of the biocompatible material is more compact, making the ophthalmic drainage tube less prone to rupture, leakage, or dissolution and more stable.

The imine group may be measured by a method commonly used in the art: taking an ophthalmic drainage device with a mass of m, and measuring the molar content n0 of the imine group by infrared or nuclear magnetic resonance, where the molar content of the imine group per unit mass of the biocompatible material may be n0/m.

In some implementations, the biocompatible material in the ophthalmic drainage device contains 0.3 to 0.5 micromole of the ester group per milligram.

The ester group may be measured by the following method: taking an ophthalmic drainage device with a mass of m, and measuring the ester group with a biuret reagent kit.

In some embodiments, the biocompatible material further includes one or more of hydroxypropyl methylcellulose, glucose glycosaminoglycan, polylactic acid, collagen, polyglycolic acid, and hyaluronic acid, which are used for the tube wall of the ophthalmic drainage device to enhance its flexibility and provide appropriate mechanical properties. 1% to 10% of the above materials may be added to type A gelatin or type B gelatin for preparation.

### Preparation method for an ophthalmic drainage device

In a second aspect, some embodiments of the present application provide a preparation method for an ophthalmic drainage device, including:
S100, placing a raw drainage tube including a core mold in a first organic solution containing a monomer containing an aldehyde group, so that an amino group in the drainage tube is enabled to react with the aldehyde group to obtain a first drainage tube, where the raw drainage tube includes a tube wall and a drainage channel defined by the tube wall, the tube wall includes a multifunctional polymer containing hydrophilic groups and the amino group, and the hydrophilic groups include a carboxyl group and a hydroxyl group; and
S200, placing the first drainage tube in a second alkaline organic solution containing a monomer containing an epoxy group, so that the reaction of the epoxy group is enabled to obtain the ophthalmic drainage device in the first aspect.

According to the embodiments of the present application, the monomer containing the aldehyde group is cross-linked with the raw drainage tube made of the multifunctional monomer containing the hydrophilic groups and the amino group, thereby increasing the cross-linking strength of the tube wall material of the raw drainage tube; and the reaction of the monomer containing the epoxy group in the second alkaline organic solution further increases the cross-linking strength of the tube wall, improves the stability of the ophthalmic drainage device, and reduces the swelling property of the ophthalmic drainage device.

In some embodiments, the monomer containing the aldehyde group is a monofunctional monomer containing an aldehyde group. In some implementations, the monomer containing the epoxy group is a monofunctional monomer containing an epoxy group. The monofunctional monomers may avoid introducing other functional groups that affect the performance of the ophthalmic drainage tube.

The multifunctional polymer containing the hydrophilic groups and the amino group includes one or both of type A gelatin and type B gelatin. According to the embodiments of the present application, the gelatin may be a product of partial hydrolysis of collagen, belongs to a type of protein, and is mostly formed by intertwining three polypeptide chains, where the polypeptide is a mixture composed of amino acid units, with a CAS number 9000-70-8. The gelatin prepared by an acid method is type A gelatin, and the gelatin prepared by an alkali method is type B gelatin.

The monomer containing the aldehyde group includes one or more of glutaraldehyde, oxidized glucan, glyoxal, malondialdehyde, butanedial, and hexanedial;
The monomer containing the epoxy group includes one or more of polyethylene glycol diglycidyl ether (DGEG) and ethylene glycol diglycidyl ether, and optionally, polyethylene glycol diglycidyl ether with a molecular weight less than 600. The polyethylene glycol diglycidyl ether within the molecular weight range enables better cross-linking in the surface of the ophthalmic drainage tube and even the cross-linking pores of the tube wall, resulting in a higher relative density of the entire ophthalmic drainage tube.

The first organic solution and the second alkaline organic solution each include one or more of acetonitrile, ethanol, methanol, and tetrahydrofuran, and optionally acetonitrile. According to the embodiments of the present application, the first organic solution and the second alkaline organic solution are used to further increase the cross-linking strength in the tube wall and increase the relative density of the ophthalmic drainage device.

The second alkaline organic solution includes one or more of N,N-diisopropylethylamine, triethylamine, pyridine, sodium hydroxide, and potassium hydroxide. According to the embodiments of the present application, the alkaline environment can promote the reaction between the epoxy group and the carboxyl group or amino group to enhance the cross-linking degree of the biocompatible material in the tube wall.

According to the embodiments of the present application, the copolymer composed of the above substances can increase the mechanical strength of the ophthalmic drainage device, and make the network structure more compact, thereby improving the comprehensive performance of the ophthalmic drainage device.

In some implementations, the preparation method includes at least one of the following conditions:
In some implementations, the monomer with the epoxy group has an epoxy value of 0.35 to 0.91 eq/100 g. According to the embodiments of the present application, the monomer containing the epoxy group, having the epoxy value within the above range, can react with more carboxyl groups to increase cross-linking sites of the biocompatible material. On the other hand, the epoxy group can react with free amino groups to further increase cross-linking sites and improve the stability of the biocompatible material.

In some embodiments, in S100, a preparation method for the raw drainage tube including the core mold includes: placing the core mold in an aqueous solution of the multifunctional monomer containing the hydrophilic groups and the amino group to prepare the raw drainage tube including the core mold. In some embodiments, the core mold is placed in the aqueous solution of the multifunctional monomer containing the hydrophilic groups and the amino group at 30°C to 60°C to prepare the raw drainage tube including the core mold. Optionally, when the aqueous solution is cooled to 0°C to 4°C, the raw drainage tube including the core mold is obtained.

In some embodiments, in S100, a preparation method for the raw drainage tube including the core mold includes: placing the aqueous solution of the multifunctional monomer containing the hydrophilic groups and the amino group in a mold to prepare the raw drainage tube including the core mold. In some embodiments, the aqueous solution of the multifunctional monomer containing the hydrophilic groups and the amino group at 30°C to 60°C is placed in the mold to prepare the raw drainage tube including the core mold. Optionally, when the aqueous solution is cooled to 0°C to 4°C, the raw drainage tube is obtained.

In some embodiments, in S100, the monomer containing the aldehyde group is glutaraldehyde. Normally, the commercially available glutaraldehyde solution is an aqueous solution of glutaraldehyde. In some embodiments, a preparation method for a glutaraldehyde organic solution includes mixing the aqueous solution of glutaraldehyde with a first organic solvent and a solid moisture desiccant to prepare the glutaraldehyde organic solution. As an example, 20 ml of 50% (w/v) glutaraldehyde solution is added to 480 ml of acetonitrile solution, 100 g of solid moisture desiccant is added to the mixed solution, which is then sealed and dried for 0.5 hour, and the solid desiccant is filtered out with a Buchner funnel to obtain the glutaraldehyde organic solution.

In some embodiments, in S100, the first drainage tube has an average cross-linking degree of 80% to 97%. A testing method for the cross-linking degree may be well known in the art. As an example, a TNBS (2,4,6-trinitrobenzenesulfonic acid) method may be used: adding 1 ml of 5% (w/v) TNBS solution and 1 ml of 4% (w/v) sodium bicarbonate solution to 3-5 mg (m1) of a cross-linked sample, heating the mixed solution to 40°C and maintaining the temperature for 2 hours, adding 2 ml of 6 mol/L hydrochloric acid solution, heating the mixed solution again to 60°C and maintaining the temperature for 1.5 hours, cooling the mixed solution to 25°C, measuring absorbance A1 of the sample at 410 nm, measuring absorbance A0 of an uncross-linked sample with a mass of m0 by the same method, and obtaining the cross-linking degree (%) = 1 - (A1/m1)/ (A0/m0).

In some implementations, in S200, after a first drainage tube containing a ketone group is obtained, the preparation method further includes: removing the unreacted monomer containing the aldehyde group.

In some embodiments, in S200, removing the unreacted monomer containing the aldehyde group includes: placing the first drainage tube in a cellulose dialysis membrane, and removing the unreacted monomer containing the aldehyde group in a buffer solution. In some embodiments, the buffer solution includes one or more of phosphate buffer solution, deionized water, and PBS solution.

According to the embodiments of the present application, the unreacted monomer containing the aldehyde group may be removed with a cellulose dialysis membrane or a cellulose dialysis membrane with molecular weight cut-off (Mw) of 20 KD, thereby reducing voids in the first drainage tube and increasing network density.

In some embodiments, in S200, after the first drainage tube is placed in a second alkaline organic solution containing a monomer containing an epoxy group, so that the carboxyl group is enabled to react with the epoxy group, the method includes: removing the unreacted monomer containing the epoxy group.

In some embodiments, in S200, removing the unreacted monomer containing the epoxy group includes: placing the first drainage tube in a cellulose dialysis membrane, and removing the unreacted monomer containing the epoxy group in a buffer solution. In some embodiments, the buffer solution includes one or more of phosphate buffer solution, deionized water, and PBS solution.

According to the embodiments of the present application, the unreacted monomer containing the epoxy group may be removed with a cellulose dialysis membrane or a cellulose dialysis membrane with molecular weight cut-off (Mw) of 20 KD.

According to the embodiments of the present application, the method is simple, the production conditions are mild, and the prepared ophthalmic drainage tube has good mechanical properties and biocompatibility; the cross-linking site in the cross-linking network is directly connected to the monomer containing the aldehyde group (including gelatin), and the organic solvent (including acetonitrile) is used as a reaction environment instead of water to limit structural looseness of the material during cross-linking, making the cross-linked structure more compact and the relative density of the product obtained after cross-linking higher. After cross-linking occurs in S100, the voids of the polymer are small, but the small molecular weight DGEG can still penetrate into the material. In the secondary cross-linking in S200, the monomer containing the epoxy group (including DGEG) reacts with the carboxyl group (-COOH) and hydroxyl group (-OH) of gelatin molecules, resulting in a higher relative density of the gelatin molecules, thereby greatly slowing down the rupture, leakage, or dissolution process of the ophthalmic drainage device in vivo. The ophthalmic drainage device of the present application is degradable and can remain stable in vivo for a long time, and therefore, has a greater practical value.

### Embodiments

The following embodiments more specifically describe the content disclosed in the present application, and these embodiments are only used for explanatory description, because various modifications and changes made within the scope of the content disclosed in the present application are apparent to those skilled in the art. Unless otherwise stated, all parts, percentages, and ratios described in the following embodiments are based on weight, all reagents used in the embodiments are commercially available or synthesized according to conventional methods and can be directly used without further treatment, and all instruments used in the embodiments are commercially available.

### Embodiment 1

This embodiment provides a preparation method for an ophthalmic drainage device, including:

25 g of gelatin (commercially available type A gelatin from pig skin, its amino content may be measured by a TNBS method with glycine as a standard curve, and its carboxyl content may be inferred to be greater than 0.4 µmol/mg based on the amino content and the acidity of a gelatin solution) containing a carboxyl group, a hydroxyl group, and an amino group was stirred into 100 ml of PBS solution to obtain a gelatin solution, which was then heated at 55°C until the gelatin was completely dissolved, and the gelatin solution was ultrasonically degassed. The temperature was maintained at 55°C and the gelatin solution was injected into a mold. The gelatin solution was slowly cooled to 4°C to prepare a plurality of tubular materials, and the molded materials were taken out to obtain raw drainage tubes.

20 ml of 50% (w/v) glutaraldehyde solution was added to 480 ml of acetonitrile solution, 100 g of solid moisture desiccant was added to the mixed solution, which was then sealed and dried for 0.5 hour, and the solid desiccant was filtered out with a Buchner funnel to obtain a 2% glutaraldehyde organic solution.

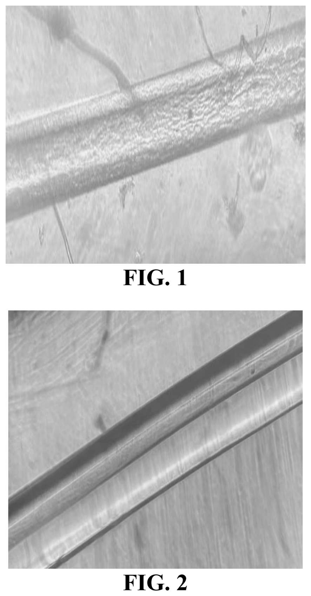

The raw drainage tube was mixed with the glutaraldehyde organic solution to obtain a mixture, which was then placed in a shaking incubator and reacted for 20 hours under shaking while the temperature was controlled at 25°C to obtain a first drainage tube. The first drainage tube was taken out, placed in a cellulose dialysis membrane with molecular weight cut-off (Mw) of 20 KD, and dialyzed in 2 L of PBS solution for 20 hours, with the dialysis solution changed every 4 hours during this period. After dialysis, a first drainage tube was obtained. The first drainage tube was mixed with a mixed solution including 10 ml of ethylene glycol diglycidyl ether, 0.5 ml of diisopropylethylamine, and 40 ml of acetonitrile to obtain a mixture, which was then placed in the 25°C shaking incubator and reacted for 20 hours under shaking to obtain a cross-linked ophthalmic drainage tube. The cross-linked ophthalmic drainage tube was dialyzed in 2 L of PBS solution for 20 hours, with the dialysis solution changed every 4 hours during this period. After dialysis, an ophthalmic drainage tube was obtained. The ophthalmic drainage tube prepared in Embodiment 1 was observed under a scanning electron microscope, and results shown in FIG. 1 were obtained, indicating that the ophthalmic drainage tube had a compact network structure.

### Embodiment 2

This embodiment differs from Embodiment 1 in that 10 ml of ethylene glycol diglycidyl ether was changed to 10 ml of polyethylene glycol diglycidyl ether with a molecular weight of 218.25 (Mw) and an epoxy value of 0.91 mol/100 g.

### Embodiment 3

This embodiment differs from Embodiment 1 in that ethylene glycol diglycidyl ether was replaced with polyethylene glycol diglycidyl ether with an epoxy value of 0.35 to 0.40 eq/100 g, and the polyethylene glycol diglycidyl ether was a mixture with a molecular weight (Mw) of 482 to 571.

### Embodiment 4

This embodiment differs from Embodiment 1 in that: 25 g of gelatin was mixed with 0.25 g of a mixture of hydroxypropyl methyl cellulose, glucose glycosaminoglycan, and polylactic acid (a mass ratio of hydroxypropyl methyl cellulose: glucose glycosaminoglycan: polylactic acid was 1: 1: 1) (the gelatin was commercially available type A gelatin from pig skin, its amino content may be measured to be 0.25 to 0.4 µmol/mg by a TNBS method with glycine as a standard curve, and its carboxyl content may be inferred to be greater than 0.4 µmol/mg based on the amino content and the acidity of a gelatin solution), where the gelatin contained a carboxyl group, a hydroxyl group, and an amino group. The gelatin mixed with the mixture was stirred into 100 ml of PBS solution to obtain a gelatin solution, which was then heated at 55°C until the gelatin was completely dissolved, and the gelatin solution was ultrasonically degassed. The temperature was maintained at 55°C and the gelatin solution was injected into a mold. The gelatin solution was slowly cooled to 4°C to prepare a plurality of tubular materials and obtain raw drainage tubes. The rest was the same as Embodiment 1.

### Comparative Example 1

This comparative example differs from Embodiment 1 in that: The raw drainage tube was mixed with a 2% glutaraldehyde organic solution, and the 2% glutaraldehyde organic solution did not contain water. The first drainage tube did not undergo subsequent reactions, and an ophthalmic drainage tube with the same moisture content as Embodiment 1 was prepared.

### Comparative Example 2

This comparative example differs from Embodiment 1 in steps: the raw drainage tube was mixed with a glutaraldehyde organic solution to obtain a mixture, which was then placed in a shaking incubator and reacted for 20 hours under shaking while the temperature was controlled at 25°C to obtain a first drainage tube, where the acetonitrile in the glutaraldehyde organic solution was replaced with pure water, i.e., the raw drainage tube was mixed with an aqueous solution of glutaraldehyde, and a moisture desiccant was not required for drying.

### Comparative Example 3

This comparative example differs from Embodiment 1 in steps: the raw drainage tube was mixed with an aqueous solution of glutaraldehyde to obtain a mixture, which was then placed in a shaking incubator and reacted for 20 hours under shaking while the temperature was controlled at 25°C to obtain a first drainage tube. Compared with Embodiment 1, the acetonitrile in the glutaraldehyde organic solution was replaced with pure water, i.e., the raw drainage tube was mixed with the aqueous solution of glutaraldehyde, a moisture desiccant was not required for drying, the first drainage tube did not undergo subsequent reactions, and an ophthalmic drainage tube with the same moisture content as Embodiment 1 was prepared.

### Test section

### Microstructure observation:

The ophthalmic drainage tubes prepared in Embodiment 1 and Comparative Example 2 were observed under a microscope, and 400-fold microscopic images were obtained. FIG. 1 shows a microstructure of the ophthalmic drainage tube in Comparative Example 2, and FIG. 2 shows a microstructure of the ophthalmic drainage tube in Embodiment 1. It can be seen from the figures that the ophthalmic drainage tube in Embodiment 1 has a more compact tube wall structure than that in Comparative Example 2.

Leakage test of the drainage tubes: The ophthalmic drainage tubes prepared in the embodiments and the comparative examples were loaded into a flow pump pipeline at room temperature, with both ends connected; the flow rate was set to 0.04 ml/min, and the leakage statuses on the surfaces of the drainage devices were observed:

Details are as shown in Table 1.

**Table 1**

| Embodiment | Leakage time |
|---|---|
| Embodiment 1 | No leakage within 12 hours |
| Embodiment 2 | No leakage within 12 hours |
| Embodiment 3 | No leakage within 12 hours |
| Comparative Example 1 | Leakage starting from 5 hours |
| Comparative Example 2 | Leakage starting from 10 hours |
| Comparative Example 3 | Leakage starting from 2 hours |

From the above table, it can be seen that the dual-network drainage devices in Embodiments 1-3 did not show any leakage within 14 days. Compared with Comparative Example 1 and Comparative Example 3, acetonitrile was used as a reaction solution instead of water to significantly increase the relative density of the drainage device.

From Table 1, when the acetonitrile was used as a cross-linking agent instead of water, the final relative density of the tube was greatly improved; and the addition of the monomer containing the epoxy group also limited fluffiness. Reasons are as follows: the reaction between the amino group (-NH₂) in the gelatin and the glutaraldehyde to form a Schiff base was a relatively slow process; during reaction, the gelatin was cross-linked with the reaction to establish a network and continuously swelled until a reaction equilibrium was established; when the acetonitrile was used as a reaction solvent instead of water, as the amino group (-NH₂) in the gelatin reacted with the glutaraldehyde to form a Schiff base network, the gelatin did not swell or swelled slightly during cross-linking in the absence of water, resulting in a more compact network structure of the product; and after the reaction of the ethylene glycol diglycidyl ether with the carboxyl group (-COOH) and hydroxyl group (-OH) of the gelatin to form a cross-linked network, the swelling of the gelatin was limited again.

Compared with Examples 1-3, in Comparative Examples 2 and 3, the monomers were cross-linked in an aqueous solution environment instead of an organic solvent environment, resulting in relatively large voids and a relatively loose network structure of the prepared ophthalmic drainage device, and ultimately leading to a loose network structure and lower relative density of the product.

In Comparative Example 1, only two types of monomers were used for cross-linking, and no monomers containing epoxy ring structures were used for secondary cross-linking, resulting in a relatively low cross-linking degree and a relatively loose network structure of the prepared ophthalmic drainage device, thereby making the relative density of the product lower.

Measurement of relative density: two side lengths and a thickness of each of the ophthalmic drainage tube samples in the embodiments and the comparative examples were accurately measured with a vernier caliper, a volume (V1) of a stent was calculated, and each sample was weighed (W1), immersed in an anhydrous ethanol solution until saturation, taken out and weighed (W2), where the density of anhydrous ethanol was denoted as ρ, and the relative density of the ophthalmic drainage tube P (%) = 1 - (W2 - W1)/ (ρ • V1) × 100%. The results were shown in Table 2. The LB 100 microscope of Guangzhou Laite Photoelectric Technology Co., Ltd. was used to observe the tube wall of the ophthalmic drainage tube in Embodiment 1, and results shown in FIG. 1 were obtained. A microstructure can be observed from the figure, indicating that the network structure in the tube wall was compact, with a high relative density.
P: Relative density of the ophthalmic drainage tube sample, in %;
W2: Mass of the ophthalmic drainage tube sample immersed in ethanol until saturation, in grams (g);
W1: Mass of the ophthalmic drainage tube sample before being immersed in ethanol, in grams (g);
ρ: Density of anhydrous ethanol, 0.79 g/cm³;
V1: Volume of the ophthalmic drainage tube sample, in cm³.

**Table 2**

| Embodiment | Relative density/% |
|---|---|
| Embodiment 1 | 60 |
| Embodiment 2 | 58 |
| Embodiment 3 | 44 |
| Comparative Example 1 | 35 |
| Comparative Example 2 | 32 |
| Comparative Example 3 | 30 |

From the above table, the relative density in Embodiments 1-3 was more than 44%, while the relative density in Comparative Example 3 was relatively low, indicating insufficient cross-linking. The relative density in Comparative Examples 1 and 2 was relatively high, but lower than that in the embodiments. Consequently, the ophthalmic drainage tube in the embodiments had a higher relative density, smaller material voids, a higher network density, higher compressive strength, and higher stability.

In vitro simulation experiment: the drainage device was installed on a cellulose membrane, the flow rate was controlled at 7 µl/min with a cross flow pump, a PBS solution was circulated, and the rupture time of the drainage device was recorded, as shown in Table 3:

**Table 3**

| Embodiment | In vitro simulation experiment | |
|---|---|---|
| | Weight loss starting time (days) | Total degradation time (days) |
| Embodiment 1 | 12 | No rupture within 60 days |
| Embodiment 2 | 11 | No rupture within 60 days |
| Embodiment 3 | 12 | No rupture within 60 days |
| Comparative Example 1 | 10 | Rupture starting from 12 days |
| Comparative Example 2 | 1 | Rupture starting from 50 days |
| Comparative Example 3 | 1 | Rupture starting from 10 days |

From the results of the in vitro simulation experiment in Table 3, it can be seen that the dual-network cross-linked drainage devices in all the embodiments and the comparative examples exhibited superior stability compared to single-network drainage devices.

Test of mechanical properties: Compressive stress of the ophthalmic drainage tubes prepared in the embodiments and the comparative examples: the ophthalmic drainage tube was fixed on a servo computer desk type tensile testing machine (Suzhou Tophung Machinery Co., Ltd.) and uniformly pressed along the length of the tube wall, and the force on the tube wall after being pressed down by 0.15 mm was measured.

Shear force: the ophthalmic drainage tube was fixed on the servo computer desk type tensile testing machine, and a maximum force value in the shearing process was measured as shear force.

Axial push: whether the ophthalmic drainage tube passed through a U-shaped tube seat smoothly was tested on a U-shaped fixture of the servo computer desk type tensile testing machine (Suzhou Tophung Machinery Co., Ltd.).

Flexibility: the ophthalmic drainage tube was fixed on an analytical balance (Sartorius) and uniformly pressed along the length of the tube wall, and the balance was read after the tube was compressed by 0.04 mm. After the material was compressed to a certain extent in the flexibility test, the elastic mass (in grams) of the material on the electronic balance was measured, and the tested mass value was converted into an elastic value (N).

**Table 4**

| Embodiment | Compressive stress | Flexibility | Shear force | Axial push |
|---|---|---|---|---|
| Embodiment 1 | 1.65 N | 0.65 N | 0.91 N | Pass |
| Embodiment 2 | 1.51 N | 0.53 N | 0.85 N | Pass |
| Embodiment 3 | 1.49 N | 0.59 N | 0.87 N | Pass |
| Comparative Example 1 | 0.33 N | 0.49 N | 0.2 N | Fail |
| Comparative Example 2 | 0.66 N | 0.47 N | 0.53 N | Pass |
| Comparative Example 3 | 0.12 N | 0.41 N | 0.09 N | Fail |

From the mechanical property results in Table 4, it can be seen that the dual-network ophthalmic drainage tube in the embodiments had higher compressive strength and higher flexibility than those in the comparative examples and can pass through the U-shaped tube seat within a compression range.

Described above are merely specific implementations of the present application, but the scope of protection of the present application is not limited thereto. Any skilled person who is familiar with this art can readily conceive of various equivalent modifications or substitutions within the disclosed technical scope of the present application, and these modifications or substitutions shall fall within the scope of protection of the present application. Therefore, the protection scope of the present application shall be subject to the scope of protection of the claims.

## Claims

1. An ophthalmic drainage device, comprising: a tube wall and a drainage channel defined by the tube wall, wherein
the tube wall comprises a biocompatible material comprising a polymer containing an ether group, an ester group, and an imino group;
the biocompatible material in the ophthalmic drainage device has an average cross-linking degree of 60% to 90%; and
in a 25°C anhydrous ethanol solution, the ophthalmic drainage device has a relative density of 40% to 60%.

2. The ophthalmic drainage device according to claim 1, wherein the biocompatible material in the ophthalmic drainage device contains 0.25 to 0.4 micromole of the imine group per milligram.

3. The ophthalmic drainage device according to claim 1, wherein the biocompatible material in the ophthalmic drainage device contains 0.3 to 0.5 micromole of the ester group per milligram.

4. The ophthalmic drainage device according to any one of claims 1 to 3, wherein the biocompatible material further comprises at least one of hydroxypropyl methylcellulose, glucose glycosaminoglycan, polylactic acid, collagen, polyglycolic acid, or hyaluronic acid.

5. A preparation method for an ophthalmic drainage device, comprising:
placing a raw drainage tube comprising a core mold in a first organic solution containing a monomer containing an aldehyde group, so that an amino group in the drainage tube is enabled to react with the aldehyde group to obtain a first drainage tube, wherein the raw drainage tube comprises a tube wall and a drainage channel defined by the tube wall, the tube wall comprises a multifunctional polymer containing hydrophilic groups and the amino group, and the hydrophilic groups comprise a carboxyl group and a hydroxyl group; and
placing the first drainage tube in a second alkaline organic solution containing a monomer containing an epoxy group, so that the reaction of the epoxy group is enabled to obtain the ophthalmic drainage device according to claim 1 or 2.

6. The preparation method according to claim 5, wherein the preparation method comprises at least one of the following conditions:
the multifunctional polymer containing the hydrophilic groups and the amino group comprises one or both of type A gelatin and type B gelatin;
the monomer containing the aldehyde group comprises at least one of glutaraldehyde, oxidized glucan, glyoxal, malondialdehyde, butanedial, hexanedial/o-phthalaldehyde, isophthalaldehyde, terephthalaldehyde, 2,3-naphthalenediacetal 2,5-diformylfuran, 2,5-dimethoxybenzene-1,4-dialdehyde, 4-tert-butyl-2,6-formylphenol, biphenyl-2,2-dialdehyde, 1,4-cyclohexanedimethanol glycidyl ether, resorcinol diglycidyl ether, pentaerythritol glycidyl ether, or neopentyl glycol diglycidyl ether;
the monomer containing the epoxy group comprises at least one of polyethylene glycol diglycidyl ether, ethylene glycol diglycidyl ether/terminal epoxy modified polyethylene glycol, terminal amino modified polyethylene glycol or ethylene glycol, aminated glucan, 1,4-butanediol glycidol, or diglycidyl ether;
the first organic solution and the second alkaline organic solution each comprise at least one of acetonitrile, ethanol, methanol, or tetrahydrofuran; or
the second alkaline organic solution comprises at least one of N,N-diisopropylethylamine, triethylamine, pyridine, sodium hydroxide, or potassium hydroxide.

7. The preparation method according to claim 5, wherein the first drainage tube has an average cross-linking degree of 80% to 97%.

8. The preparation method according to claim 5, wherein the monomer containing the epoxy group has an epoxy value of 0.35 to 0.91 eq/100 g.

9. The preparation method according to claim 5, wherein the first drainage tube contains a ketone group, and after the placing a raw drainage tube comprising a core mold in a first organic solution containing a monomer containing an aldehyde group, so that an amino group in the drainage tube is enabled to react with the aldehyde group to obtain a first drainage tube, the preparation method further comprises:
removing the unreacted monomer containing the aldehyde group.

10. The preparation method according to claim 5, wherein after placing the first drainage tube in a second alkaline organic solution containing a monomer containing an epoxy group, so that the carboxyl group is enabled to react with the epoxy group, the preparation method comprises:
removing the unreacted monomer containing the epoxy group.
